# EUROPEAN PATENT APPLICATION

(11) **EP 4 339 522 A1**
(43) Date of publication of application: **20.03.2024**
(21) Application number: 23197259.7
(22) Date of filing: 13.09.2023
(51) Int. Cl.: F24F 8/22

(54) **AIR CONDITIONER**

(30) Priority: 14.09.2022 KR 20220115325
(71) Applicant: LG Electronics Inc., Yeongdeungpo-gu Seoul 07336 (KR)
(72) Inventor: JEONG, Suhyeon, Seoul (KR); SHIN, Sooyeon, Seoul (KR); PARK, Geunyoung, Seoul (KR)
(74) Representative: Vossius & Partner Patentanwälte Rechtsanwälte mbB

(57) **Abstract**

The present disclosure relates to an air conditioner. The air conditioner according to an embodiment of the present disclosure includes: a case having an inlet and an outlet; a heat exchanger disposed inside the case; a fan disposed inside the case and elongated in a direction of a rotation axis; a guide extending toward the outlet in a rotation direction of the fan; and a sterilizer mounted to the guide at a position facing the fan, and provided with a lamp extending in a direction in which the fan extends, thereby providing a uniform sterilizing effect in the longitudinal direction of the fan.

## Description

An air conditioner, more particularly, to an air conditioner capable of sterilizing a fan.

An air conditioner is a device for exchanging heat with air and supplying the heatexchanged air to a room.

An air-blowing fan is mounted inside a case of the air conditioner. The fan causes indoor air to be drawn into the case through an inlet, and blows the drawn air into the room through an outlet.

Recently, a fan occupying a large volume in the air conditioner is generally used in order to increase cooling and heating capacity and air-blowing capacity.

However, an existing air conditioner has a problem in that the entire large area of the fan is contaminated with foreign matter contained in the intake air.

In addition, the existing air conditioner also has a problem in that in order to sterilize or disinfect the entire area of the fan occupying a large space, a plurality of light sources are required, increasing the production costs, and making it difficult to uniformly sterilize the entire area of the fan.

Furthermore, the existing air conditioner also has a problem in that a light source module disposed in a passage for sterilizing a fan causes resistance to the airflow.

The invention is specified by the independent claim. Preferred embodiments are defined by the dependent claims. It is an objective of the present disclosure to solve the above and other problems.

It is another objective of the present disclosure to sterilize a fan.

It is yet another objective of the present disclosure to uniformly sterilize a large area of a fan.

It is yet another objective of the present disclosure to simplify a structure of a sterilizer.

It is further another objective of the present disclosure to eliminate the flow resistance.

It is further another objective of the present disclosure to simplify the assembly of a sterilizer.

The objectives of the present disclosure are not limited to the aforementioned objectives and other objectives not described herein will be clearly understood by those skilled in the art from the following description.

In accordance with an aspect of the present disclosure, the above and other objectives can be accomplished by providing an air conditioner including a case having an inlet and an outlet.

The air conditioner includes a heat exchanger disposed inside the case.

The air conditioner includes a fan disposed inside the case and elongated in a direction of a rotation axis.

The air conditioner includes a guide extending toward the outlet in a rotation direction of the fan.

The air conditioner includes a sterilizer mounted to the guide at a position facing the fan, and provided with a lamp extending in a direction in which the fan extends, thereby providing a uniform sterilizing effect in the longitudinal direction of the fan.

The guide may include a rear guide extending in the rotation direction of the fan.

The guide may include a front guide disposed downstream of the rear guide and extending toward the outlet.

The sterilizer may be mounted to the front guide.

The sterilizer may be coupled to the front guide to be detachable from the case.

The front guide may include a device coupling portion recessed in a direction away from the fan, and providing a space in which the sterilizer is disposed.

The sterilizer may include a lamp case in which the lamp is accommodated, and which is coupled to the front guide.

The sterilizer and the front guide may extend in the direction of the rotation axis of the fan.

The sterilizer may form a continuous surface with a surface of the front guide that faces the fan.

The case may include a front wall spaced from one side of the fan.

The front guide may include a first plate disposed under the front wall and the rear guide.

The front guide may include a first rib protruding upward from the first plate.

The front guide may include a second rib protruding upward from the first plate and spaced apart from the first rib.

The front wall and the rear guide may be inserted between the first rib and the second rib.

The front guide may include a lower protrusion protruding toward the outlet and disposed below the sterilizer.

The sterilizer may include a case cover forming a continuous surface with a surface of the rear guide that faces the fan.

The rear guide may include a guide protrusion protruding toward the fan and disposed above the sterilizer.

The rear guide may include a plurality of guide protrusions protruding toward the fan, and spaced apart from each other in the direction in which the fan extends.

The sterilizer may include holders disposed at positions between the plurality of guide protrusions.

The air conditioner may include a drain pan disposed on a lower side of the heat exchanger and disposed below the sterilizer.

The drain pan may include a base disposed on a lower side of the heat exchanger.

The drain pan may include a first drain pan wall extending from the base toward the sterilizer, and disposed below the sterilizer.

The drain pan may include a drain pan protrusion protruding from the first drain pan wall in a direction away from the sterilizer.

The sterilizer may include a light emitting portion disposed on one side of the lamp and having a light source connected to the lamp.

The air conditioner may include a fan motor disposed on one side of the fan and coupled to the fan.

The light emitting portion may be disposed at a position corresponding to the fan motor.

The sterilizer may include a plurality of pattern portions protruding toward a center of the lamp, and spaced apart from each other in a direction in which the lamp extends.

The plurality of pattern portions are spaced at intervals that are set differently in a longitudinal direction of the lamp.

The sterilizer may include a light source disposed on one side of the lamp and connected to the lamp.

The intervals between the plurality of pattern portions may become narrower away from the light source.

Other detailed matters of the exemplary embodiments are included in the detailed description and the drawings.

According to at least one of the embodiments of the present disclosure, a fan may be sterilized by providing a sterilizer facing the fan.

According to at least one of the embodiments of the present disclosure, a large area of a fan may be uniformly sterilized by using a sterilizer elongated in a direction in which the fan extends.

According to at least one of the embodiments of the present disclosure, a portion of an air guide is coupled with a sterilizer, thereby facilitating assembly of the sterilizer.

According to at least one of the embodiments of the present disclosure, a portion of an air guide and a sterilizer may be removed together from a case, thereby facilitating repair of the sterilizer.

According to at least one of the embodiments of the present disclosure, one surface of a sterilizer and one surface of an air guide form a continuous surface, thereby minimizing flow resistance caused by the sterilizer.

The effects of the present disclosure are not limited to the aforesaid, and other effects not described herein will be clearly understood by those skilled in the art from the following description of the appended claims.

### BRIEF DESCRIPTION OF THE DRAWINGS

FIG. 1 is a perspective view of an air conditioner according to an embodiment of the present disclosure.
FIG. 2 is a partial view of an air conditioner according to an embodiment of the present disclosure.
FIG. 3 is a cross-sectional view of an air conditioner according to an embodiment of the present disclosure.
FIG. 4 is a partial view of an air conditioner according to an embodiment of the present disclosure.
FIG. 5 is a partial view of an air conditioner according to an embodiment of the present disclosure.
FIG. 6 is a partial view of an air conditioner according to an embodiment of the present disclosure.
FIG. 7 is a partial view of an air conditioner according to an embodiment of the present disclosure.
FIG. 8 is a perspective view of a sterilizer according to an embodiment of the present disclosure.
FIG. 9 is a partial view of a sterilizer according to an embodiment of the present disclosure.
FIG. 10 is a partial view of a sterilizer according to an embodiment of the present disclosure.
FIG. 11 is a view conceptually illustrating a portion of an air conditioner according to an embodiment of the present disclosure.

### DETAILED DESCRIPTION OF THE PREFERRED EMBODIMENTS

Hereinafter, the present disclosure will be described in detail with reference to the accompanying drawings, in which the same reference numerals are used throughout the drawings to designate the same or similar components, and a redundant description thereof will be omitted.

The terms "module" and "unit" for elements used in the following description are given simply in view of the ease of the description, and do not have a distinguishing meaning or role.

It will be noted that a detailed description of known arts will be omitted if it is determined that the detailed description of the known arts can obscure the embodiments of the invention. Further, the accompanying drawings are used to help easily understand various technical features and it should be understood that the embodiments presented herein are not limited by the accompanying drawings. As such, the present disclosure should be construed to extend to any alterations, equivalents and substitutes in addition to those which are particularly set out in the accompanying drawings.

It will be understood that, although the terms first, second, etc., may be used herein to describe various elements, these elements should not be limited by these terms. These terms are only used to distinguish one element from another.

It will be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present.

As used herein, the singular forms are intended to include the plural forms as well, unless the context clearly indicates otherwise.

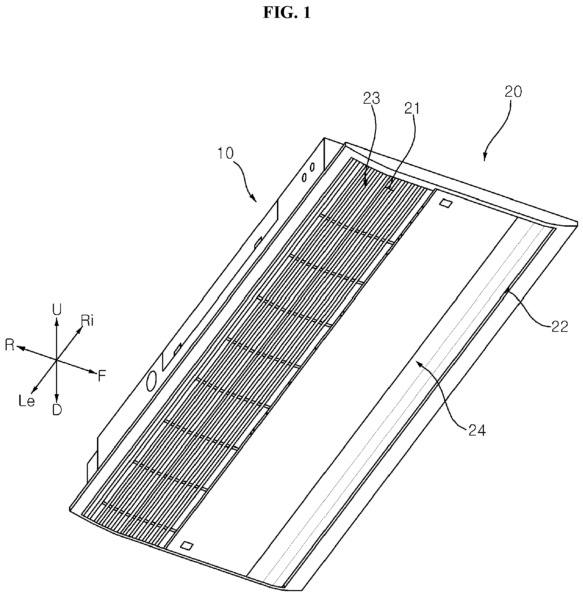

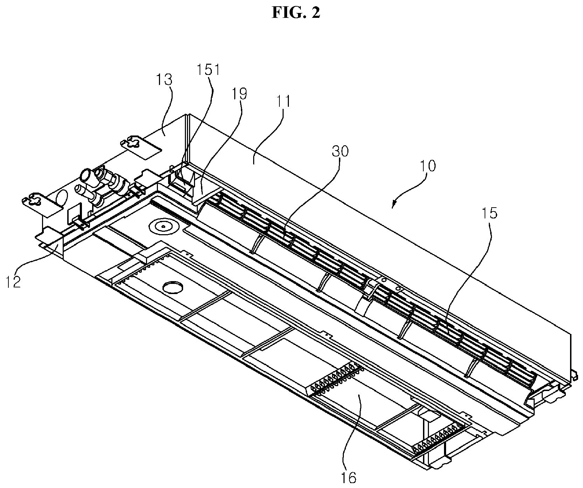

Referring to FIGS. 1 and 2, an air conditioner 1 will be described below.

FIG. 1 is a perspective view of an air conditioner 1 according to an embodiment of the present disclosure. FIG. 2 is a perspective view of the air conditioner 1, from which a cover 20 is removed.

The air conditioner 1 may include a case 10. The case 10 may be installed on the ceiling of a room. The case 10 may have an internal space.

The air conditioner 1 may include the cover 20. The cover 20 may be disposed on a lower side of the case 10. The cover 20 may have a larger cross-sectional area than the case 10.

The air conditioner 1 may include an inlet 21. The inlet 21 may be formed in the cover 20. The inlet 21 formed in the cover 20 may be open at the top and bottom. Air in the interior space may be drawn into the case 10 through the inlet 21.

The air conditioner 1 may include an outlet 22. The outlet 22 may be formed in the cover 20. The outlet 22 formed in the cover 20 may be open at the top and bottom. Air in the case 10 may be supplied to the interior space through the outlet 22.

The inlet 21 and the outlet 22 may be spaced apart from each other in a front-rear direction. The air, drawn into the case 10 through the inlet 21, may be supplied to the interior space through the outlet 22.

The air conditioner 1 may have an inlet grille 23. The inlet grille 23 may be disposed on the cover 20. The inlet 21 may be open in the inlet grille 23.

The air conditioner 1 may include a vane 24. The vane 24 may be rotatably mounted to the cover 20. The vane 24 may be rotatably mounted at the outlet 22. The vane 24 may open and close the outlet 22.

The air conditioner 1 may include a fan 15. The fan 15 may be disposed inside the case 10 of the fan 15. The fan 15 may draw in air from the outside of the case 10 and blow the air through the outlet 22.

The air conditioner 1 may include a filter 16. The filter 16 may be disposed inside the case 10. The filter 16 may be disposed on an upper side of the inlet 21.

The case 10 may include a front wall 11. The front wall 11 may be spaced forward from the fan 15. The outlet 22 may be disposed under the front wall 11.

The case 10 may include a rear wall 12. The rear wall 12 may be spaced rearward from the fan 15. The rear wall 12 may be spaced rearward from the front wall 11. The inlet 21 may be formed under the rear wall 12.

The case 10 may include a side wall 13. The side wall 13 may extend in a front-rear direction. The side wall 13 may connect the front wall 11 and the rear wall 12.

The air conditioner 1 may include a partition wall 19. The partition wall 19 may be disposed in the case 10. The partition wall 19 may be spaced apart from the side wall 13. The partition wall 19 may face the fan 15.

The air conditioner 1 may include a fan motor 151. The fan motor 151 may be coupled to the fan 15. The fan motor 151 may be disposed in the case 10. The fan motor 151 may be disposed between the side wall 13 and the partition wall 19.

The air conditioner 1 may include a front guide 30. The front guide 30 may be detached from the front wall 11. The front guide 30 may be disposed on an upper side of the outlet 22. The front guide 30 may be made of an Expanded Polystyrene (EPS) material. The front guide 30 may be a sound insulating material. The front guide 30 may be disposed between the fan 15 and the front wall 11. The front guide 30 may guide a flow direction of air blown through the outlet 22.

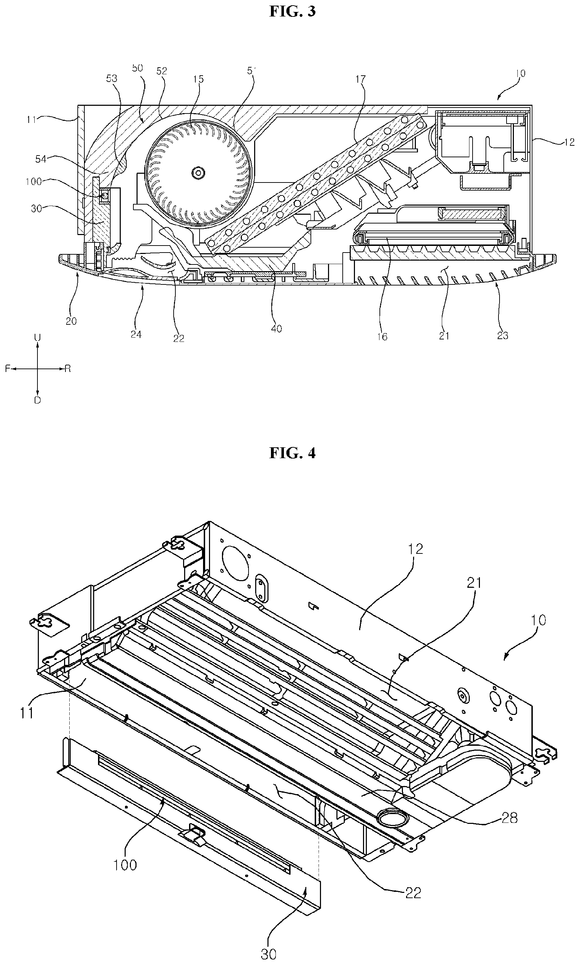

Referring to FIG. 3, the air conditioner 1 will be described below.

FIG. 3 is a vertical cross-sectional view of an air conditioner 1.

The air conditioner 1 may include a heat exchanger 17. The heat exchanger 17 may be disposed inside the case 10. The heat exchanger 17 may be an evaporator.

The air conditioner 1 may include a drain pan 40. The drain pan 40 may be disposed inside the case 10. The drain pan 40 may be disposed on a lower side of the heat exchanger 17.

The air drawn into the case 10 through the inlet 21 may be cooled by passing through the heat exchanger 17. The air, having passed through the heat exchanger 17, may be blown by the fan 15 to be discharged outside the case 10 through the outlet 22.

The heat exchanger 17 may generate condensation by heat exchanging with the air. The condensate generated in the heat exchanger 17 may be stored in the drain pan 40.

The air conditioner 1 may include a rear guide 50. The rear guide 50 may extend in a rotation direction of the fan 15. The rear guide 50 may be connected to the front guide 30. The rear guide 50 may be coupled to the front wall 11.

The air conditioner 1 may include a guide. The guide may include the rear guide 50 and the front guide 30. The guide may be a concept collectively referring to the rear guide 50 and the front guide 30.

The rear guide 50 may include a first guide 51. The first guide 51 may be disposed between the heat exchanger 17 and the fan 15. The first guide 51 may extend in the rotation direction of the fan 15.

The rear guide 50 may include a second guide 52. The second guide 52 may be disposed between the fan 15 and the front wall 11. The second guide 52 may extend in the rotation direction of the fan 15. The first guide 51 and the second guide 52 may be integrally formed.

The rear guide 50 may include a guide protrusion 53. The guide protrusion 53 may protrude toward the fan 15.

The rear guide 50 may include a third guide 54. The third guide 54 may extend in the rotation direction of the fan 15. The third guide 54 may extend downward from the second guide 52. The second guide 52 and the third guide 54 may be integrally formed. The guide protrusion 53 may protrude from the third guide 54 toward the fan 15. The third guide 54 may extend in a vertical or up-down direction, and may be connected to the front wall 11 and the cover 20. The third guide 54 may extend toward the outlet 22 and may have a space recessed toward the front wall 11.

The air conditioner 1 may include a sterilizer 100. The sterilizer 100 may be disposed to face the fan 15. The sterilizer 100 may emit ultraviolet rays toward the fan 15.

The front guide 30 may be connected to the rear guide 50. The front guide 30 may be coupled to a recessed portion of the rear guide 50. The front guide 30 is detachable from the front wall 11 and the rear guide 50. The front guide 30 may form a continuous surface with the rear guide 50.

The sterilizer 100 may be mounted to the front guide 30. The sterilizer 100 may be mounted to the front guide 30 at a position facing the fan 15. The sterilizer 100 may be disposed on a lower side of the second guide 52. The sterilizer 100 may be disposed below the guide protrusion 53. The sterilizer 100 may be disposed downstream of the rear guide 50. The sterilizer 100 may form a continuous surface with the rear guide 50 and the front guide 30.

Referring to FIG. 4, the air conditioner 1 will be described below.

FIG. 4 is a view of the air conditioner 1, from which some of the internal components of the cover 20 and the case 10 are removed.

The case 10 may include a bottom wall 28 disposed between the front wall 11 and the rear wall 12. The bottom wall 28 may be disposed between the inlet 21 and the outlet 22. The drain pan 40 (see FIG. 3) may be disposed on an upper side of the bottom wall 28. The outlet 22 may be formed between the bottom wall 28 and the front wall 11.

The front guide 30 may be inserted between the front wall 11 and the bottom wall 28. The front guide 30 may be detached from the front wall 11.

The sterilizer 100 may be coupled to the front guide 30. The sterilizer 100 may be inserted into a space formed inside the front guide 30. The sterilizer 100 may be coupled to the front wall 11 along with the front guide 30.

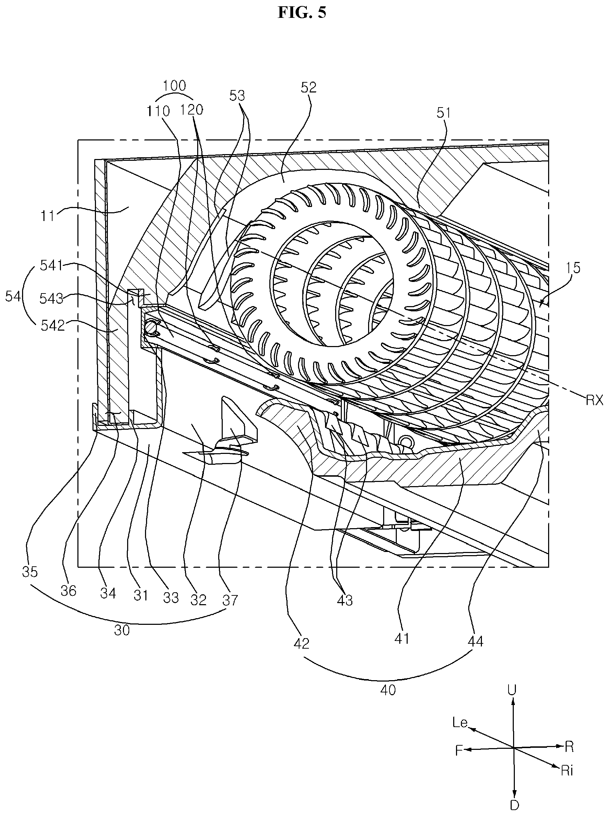

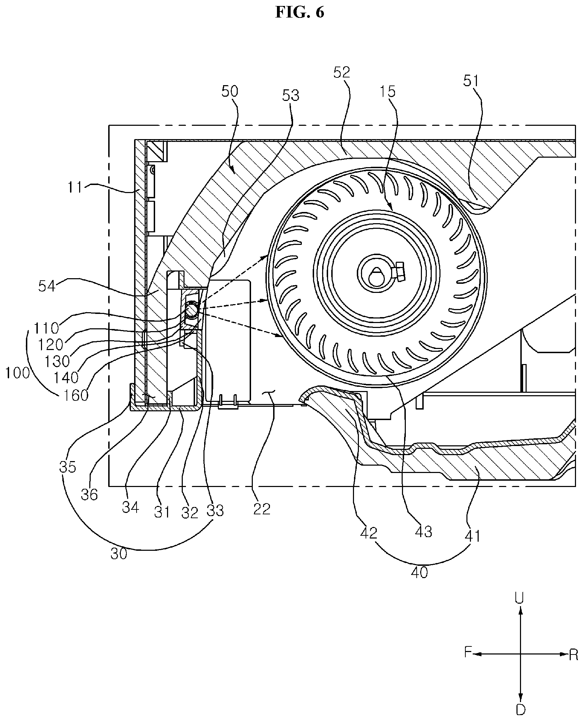

Referring to FIGS. 5 and 6, the air conditioner 1 will be described below.

FIG. 5 is a vertical sectional view of the case 10, showing an oblique view of a portion of an internal structure thereof. FIG. 6 is a vertical sectional view of the case 10, showing an internal structure thereof.

The rear guide 50 may be disposed to surround the fan 15. The first guide 51 and the second guide 52 may surround the fan 15.

The guide protrusion 53 may protrude toward the fan 15. The guide protrusion 53 may be disposed above the sterilizer 100.

The fan 15 may have a rotation axis RX. The rotation axis RX may extend in a direction orthogonal to a front-rear direction. In addition, the rotation axis RX may extend in a direction orthogonal to a vertical direction.

There may be a plurality of guide protrusions 53 that are spaced apart from each other in a direction of the rotation axis RX. The plurality of guide protrusions 53 may be spaced apart from each other in a direction in which the fan 15 extends.

The fan 15 may be a cross flow fan. The fan 15 may draw in air in a direction intersecting the rotation axis RX and may blow the air toward the outlet 22.

The third guide 54 may extend vertically. The third guide 54 may be part of the rear guide 50. The third guide 54 may be referred to as a "lower guide."

The lower guide 54 may include a first body 541 protruding downward from a lower end of the second guide 52. The first body 541 may be disposed under the guide protrusion 53. The first body 541 may face the fan 15. The first body 541 may be disposed on an upper side of the sterilizer 100.

The lower guide 54 may include a second body 542. The second body 542 may extend downward from a lower end of the second guide 52. The second body 542 may be coupled to the front wall 11. The second body 542 may be spaced forward from the first body 541. The second body 542 may be disposed between the first body 541 and the front wall 11.

The lower guide 54 may have a gap 543. The gap 543 may be formed between the first body 541 and the second body 542. The gap 543 may refer to a space formed between the second body 542 and the front guide 30.

The front guide 30 may include a first plate 31. The first plate 31 may be disposed on a lower side of the front wall 11. The first plate 31 may be disposed on a lower side of the lower guide 54. The first plate 31 may extend in a direction in which the rotation axis RX extends.

The front guide 30 may include a second plate 32. The second plate 32 may extend upward from the first plate 31. The second plate 32 may be disposed between the lower guide 54 and the fan 15. The second plate 32 may face the drain pan 40. A portion of the outlet 22 may be formed between the second plate 32 and the drain pan 40.

The front guide 30 may include a device coupling portion 33. The device coupling portion 33 may be disposed between the lower guide 54 and the fan 15. The device coupling portion 33 may be a portion recessed from the second plate 432 toward the front wall 11. The device coupling portion 33 may be coupled to the sterilizer 100. The device coupling portion 33 may be coupled to the first body 541 of the lower guide 54. The device coupling portion 33 may be inserted into the gap 543 to be fixed therein.

The front guide 30 may include a first rib 34. The first rib 34 may protrude upward from the first plate 31. The first rib 34 may be disposed between the lower guide 54 and the second plate 32.

The front guide 30 may include a second rib 35. The second rib 35 may protrude upward from the first plate 31. The second rib 35 may be spaced forward from the first rib 34.

The front guide 30 may include a groove 36. The groove 36 may be formed between the first rib 34 and the second rib 35. The first rib 34 and the second rib 35 may extend in the direction of the rotation axis RX. The groove 36 may extend in the direction of the rotation axis RX.

A lower end of the front wall 11 and a lower end of the lower guide 54 may be inserted into the groove 36. The first rib 34 and the second rib 35 may support the front wall 11 and the lower guide 54.

The front guide 30 may include a lower protrusion 37. The lower protrusion 37 may protrude from the second plate 32. The lower protrusion 37 may be disposed below the sterilizer 100. The lower protrusion 37 may be disposed below the fan 15. The lower protrusion 37 may face the drain pan 40 in a front-rear direction.

The drain pan 40 may be disposed below the fan 15. The drain pan 40 may face the front guide 30 in the front-rear direction. The drain pan 40 may extend in the direction of the rotation axis RX.

The drain pan 40 may include a base 41. The base 41 may be spaced downward from the fan 15. The condensate generated in the heat exchanger 17 (see FIG. 3) may be collected in the base 41.

The drain pan 40 may include a first drain pan wall 42. The first drain pan wall 42 may protrude upward from the base 41. The first drain pan wall 42 may extend in the direction of the rotation axis RX. The first drain pan wall 42 may be bent toward the front guide 30.

The drain pan 40 may include a drain pan protrusion 43. The drain pan protrusion 43 may be formed under the fan 15. The drain pan protrusion 43 may protrude from the first drain pan wall 42. There may be a plurality of drain pan protrusions 43 that are spaced apart from each other in the direction of the rotation axis RX. The drain pan protrusions 43 may be spaced apart from the front guide 30 in the front-rear direction. The drain pan protrusions 43 may be disposed downstream of the guide protrusion 53.

The drain pan 40 may include a second drain pan wall 44. The second drain pan wall 44 may protrude upward from the base 41. The second drain pan wall 44 may extend in the direction of the rotation axis RX. The second drain pan wall 44 may be bent toward the upstream side of the fan 15. The first drain pan wall 42 and the second drain pan wall 44 may be spaced apart from each other in the front-rear direction.

A portion of the outlet 22 may be formed between the front guide 30 and the drain pan 40. The first drain pan wall 42 and the second plate 32 may be spaced apart from each other to form the outlet 22.

The sterilizer 100 may be mounted to the front guide 30. The sterilizer 100 may be connected to the device coupling portion 33 of the front guide 30.

The sterilizer 100 may be disposed downstream of the rear guide 50. The sterilizer 100 may be disposed on a lower side of the second guide 52. The sterilizer 100 may be disposed below the guide protrusion 53.

The sterilizer 100 may be disposed to face the fan 15. The sterilizer 100 may emit ultraviolet rays toward the fan 15.

The sterilizer 100 may be disposed above the lower protrusion 37. The sterilizer 100 may be disposed under the first body 541. The sterilizer 100 may be disposed above the drain pan 40.

The sterilizer 100 may form a continuous surface with the rear guide 50 and the front guide 30. The respective surfaces of the rear guide 50, the sterilizer 100, and the front guide 30 that face the fan 15 may form a continuous surface in the rotation direction of the fan 15.

The sterilizer 100 may include a lamp 110. The lamp 110 may be elongated in the direction in which the fan 15 extends. The lamp 110 may be elongated in the direction of the rotation axis RX.

The sterilizer 100 may include a holder 120. The holder 120 may surround the lamp 110. A plurality of holders 120 may be spaced apart from each other in a direction in which the lamp 110 extends.

The sterilizer 100 may include a lamp case 130. The lamp case 130 may have an internal space. The lamp 110 may be disposed in the lamp case 130. The lamp case 130 may be disposed in the device coupling portion 33. The device coupling portion 33 may be recessed from the second plate 32 toward the front wall 11, and the lamp case 130 may be disposed in the recessed space of the device coupling portion 33. The holder 120 may protrude from the lamp case 130 toward the fan 15.

The sterilizer 100 may include a case cover 140. The case cover 140 may be coupled to the lamp case 130. The case cover 140 may be disposed between the lamp 110 and the fan 15. The case cover 140 may be made of a light-transmissive material. A surface of the case cover 140 that faces the fan 15 may form a continuous surface with one surface of the rear guide 50 and one surface of the front guide 30. The lamp 110 may be disposed in a space between the lamp case 130 and the case cover 140. Light emitted from the lamp 110 may be transmitted through the case cover 140 to be emitted toward the fan 15.

The sterilizer 100 may include a light emitting portion 160. The light emitting portion 160 may be connected to the lamp 110. The light emitting portion 160 may receive power from an external power source (not shown) to emit light. The light emitting portion 160 may be coupled to the lamp case 130. The light emitting portion 160 may be disposed at one end in the direction in which the lamp 110 extends.

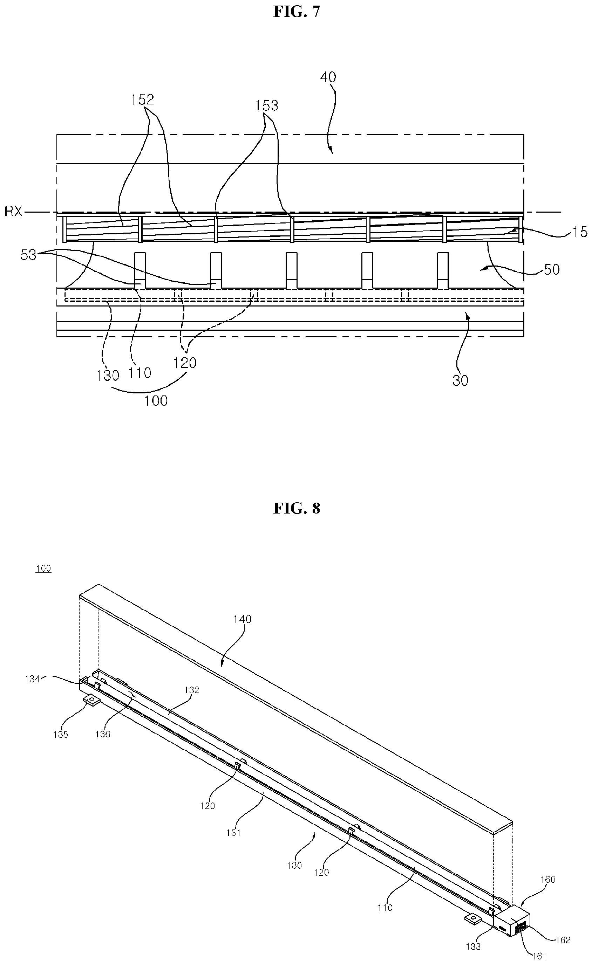

Referring to FIG. 7, the air conditioner 1 will be described below.

FIG. 7 is a view illustrating an area near the outlet 22 of the case 10, when viewed from bottom to top.

The fan 15 may have the rotation axis RX. The fan 15 may rotate about the rotation axis RX.

The fan 15 may include a plurality of blades 152. The plurality of blades 152 may extend in the direction of the rotation axis RX.

The fan 15 may include a plurality of partition plates 153. The plurality of partition plates 153 may be disposed between the blades 152.

The plurality of guide protrusions 53 may be spaced apart from each other in the direction of the rotation axis RX. The guide protrusions 53 may protrude from the rear guide 50 toward the fan 15. The respective guide protrusions 53 may be disposed at positions corresponding to the partition plates 153. The plurality of guide protrusions 53 and the plurality of partition plates 153 may face each other in the front-rear direction.

The sterilizer 100 may include the lamp 110 extending in the direction of the rotation axis RX. The lamp 110 may extend in the direction in which the fan 15 extends. The lamp 110 may uniformly project light in the direction in which the fan 15 extends. Accordingly, the light projected from the lamp 110 may be uniformly emitted over the entire area of the fan 15. The light emitted from the lamp 110 toward the fan 15 may sterilize the fan 15.

The sterilizer 100 may include the lamp case 130 extending in the direction of the rotation axis RX. The lamp case 130 may be inserted into the front guide 30 to be fixed therein.

The plurality of holders 120 may be spaced apart from each other in the direction in which the lamp 110 extends. The holders 120 may be disposed between the plurality of guide protrusions 53.

Referring to FIGS. 8 to 10, the sterilizer 100 will be described below.

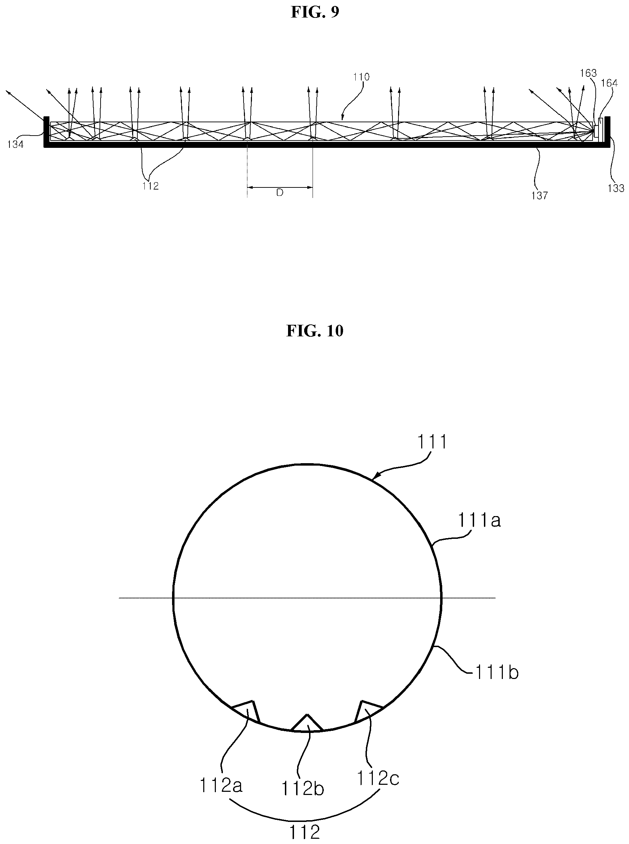

FIG. 8 is a perspective view of the sterilizer 100. FIG. 9 is a schematic diagram illustrating a traveling direction of light in the sterilizer 100. FIG. 10 is a vertical cross-sectional view of the lamp 110.

The lamp case 130 may be elongated in the direction in which the fan 15 (see FIG. 7) extends. The lamp 110 and the holders 120 may be disposed in the lamp case 130.

The lamp case 130 may include a first case wall 131. The first case wall 131 may extend in the direction in which the lamp 110 extends.

The lamp case 130 may include a second case wall 132. The second case wall 132 may extend in the direction in which the lamp 110 extends.

The first case wall 131 and the second case wall 132 may be spaced apart from each other. The lamp 110 may be disposed between the first case wall 131 and the second case wall 132.

The lamp case 130 may include a lamp receiving space 136. The lamp receiving space 136 may be a space between the first case wall 131 and the second case wall 132. The lamp 110 may be disposed in the lamp receiving space 136.

The lamp case 130 may include a first end 133. The first end 133 may connect the first case wall 131 and the second case wall 132. The first end 133 may face one end of the lamp 110.

The lamp case 130 may include a second end 134. The second end 134 may connect the first case wall 131 and the second case wall 132. The second end 134 may face another end of the lamp 110.

The lamp receiving space 136 may be formed between the first end 133 and the second end 134. The lamp 110 may extend from the first end 133 toward the second end 134.

The lamp case 130 may include coupling ribs 135. The coupling ribs 135 may protrude outside the lamp case 130. The coupling ribs 135 may be coupled to the front guide 30 (see FIG. 7).

The lamp case 130 may include a case bottom wall 137. The case bottom wall 137 may be disposed under the lamp 110. The holders 120 may protrude upward from the case bottom wall 137.

The case cover 140 may open and close the lamp receiving space 136. The case cover 140 may be removed from the lamp case 130. The case cover 140 may form a surface of the sterilizer 100 that faces the fan 15.

The sterilizer 100 may include the light emitting portion 160. The light emitting portion 160 may be coupled to one side of the lamp case 130. The light emitting portion 160 may be coupled to the first end 130.

The light emitting portion 160 may be electrically connected to an external power source (not shown). The light emitting portion 160 may generate light that is emitted toward the lamp 110.

The light emitting portion 160 may include a light source case 161. The light source case 161 may be coupled to the lamp case 130. The light source case 161 may be disposed on one side of the first end 133.

The light emitting portion 160 may include a substrate 162. The substrate 162 may receive power from the external power source (not shown). The substrate 162 may be a printed circuit board (PCB). An electric circuit may be printed on the substrate 162. The substrate 162 may be disposed in the light source case 161.

The light emitting portion 160 may include a light source 163. The light source 163 may emit light toward the lamp 110. The light source 163 may be disposed in the lamp case 130. The light source 163 may be disposed between the first end 133 and the second end 134.

The light emitting portion 160 may include a supporter 164. The supporter 164 may be coupled to the first end 133. The light source 163 may be coupled to the supporter 164. The light source 163 may pass through the first end 133 and the supporter 164 to protrude into the lamp case 130. The light source 163 may pass through the first end 133 and the supporter 164 to be electrically connected to the substrate 162.

The light source 163 may be electrically connected to the substrate 162 and may generate light by using power transmitted through the substrate 162. The light generated by the light source 163 may travel in the direction in which the lamp 110 extends. The lamp 110 may be made of a light-reflective or light-transmissive material. The light generated by the light source 163 may be reflected or refracted from or transmitted into the lamp 110 to diffuse in a longitudinal direction of the lamp 110.

The lamp 110 may include a pattern portion 112. The pattern portion 112 may be formed in the lamp 110. The pattern portion 112 may include a serration pattern. The pattern portion 112 may be formed in a protruding shape that protrudes into the lamp 110. There may be a plurality of pattern portions 112 that are spaced apart from each other in the longitudinal direction of the lamp 110. The respective pattern portions 112 may be spaced at a predetermined interval D.

The lamp 110 may include a housing 111. The housing 111 may have a cylindrical shape. The housing 111 may be made of a light-reflective or light-transmissive material.

The housing 111 may include a housing upper portion 111a and a housing lower portion 111b. The housing upper portion 111a and the housing lower portion 111b may be integrally formed.

The pattern portion 112 may be formed at a lower portion of the lamp 110. The pattern portion 112 may be formed at a position adj acent to the case bottom wall 137. The pattern portion 112 may be formed at the housing lower portion 111b.

The pattern portion 112 may be formed in a protruding shape that protrudes into the lamp 110. The pattern portion 112 may include a plurality of pattern protrusions 112a, 112b, and 112c which are spaced apart from each other around a circumference of the lamp 110. The pattern protrusions 112a, 112b, and 112c may include a first pattern protrusion 112a, a second pattern protrusion 112b, and a third pattern protrusion 112c. The respective first, second, and third pattern protrusions 112a, 112b, and 112c may protrude toward the center of the lamp 110.

The light emitted from the light source 163 toward the lamp 110 may be refracted, reflected, or transmitted within the housing 111. In this case, the pattern portion 112 may facilitate refraction or reflection of light, thereby facilitating light diffusion in the longitudinal direction of the lamp 110. There may be a plurality of pattern portions 112 that are spaced apart from each other in the longitudinal direction of the lamp 110, and the light emitted from the light source 163 may be diffused in the longitudinal direction of the lamp 110 by the plurality of pattern portions 112.

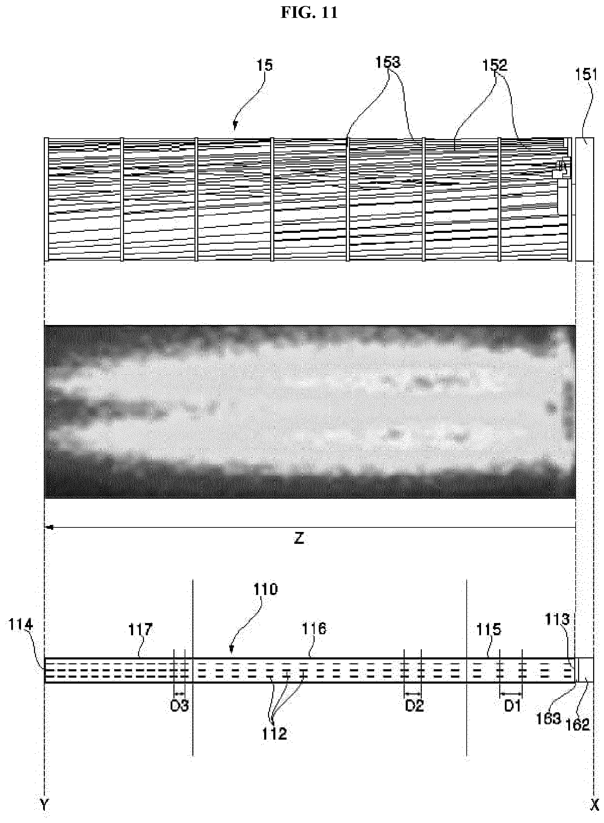

Referring to FIG. 11, the air conditioner 1 will be described below.

FIG. 11 is a view conceptually illustrating a relative arrangement between the fan 15 and the sterilizer 100 and a light diffusion effect.

In the following description, a first side on which the fan motor 151 is disposed will be defined as a first position X. In addition, a second side which is spaced apart from the fan motor 151 in the extending direction of the fan 15 will be defined as a second position Y Further, a direction from the first position X toward the second position Y will be defined as a traveling direction Z.

The fan motor 151 may be disposed at the first position X. The fan 15 may extend from the first position X toward the second position Y The blades 152 may extend in a direction from the first position X toward the second position Y The plurality of partition plates 153 may be spaced apart from each other in a direction from the first position X toward the second position Y

The substrate 162 may be disposed at the first position X. That is, the substrate 162 may be disposed at a position corresponding to the fan motor 151 in the front-rear direction, thereby facilitating management of electric parts 162 and 151 connected to the external power source (not shown). In addition, electric wires (not shown) connecting the electric parts 162 and 151 may decrease in length, and assembly may be facilitated.

The light source 163 may be disposed closer to the first position X than to the second position Y The light emitted from the light source 163 may be diffused into the lamp 110 in the traveling direction Z.

The lamp 110 may include a first lamp end 113. The first lamp end 113 may be connected to the light source 163. The first lamp end 113 may be disposed adjacent to the first position X.

The lamp 110 may include a second lamp end 114. The second lamp end 114 may be spaced apart from the first lamp end 113 in the longitudinal direction of the lamp 113. The second lamp end 114 may be disposed adjacent to the second position Y

The lamp 110 may extend from the first lamp end 113 to the second lamp end 114. The plurality of pattern portions 112 may be spaced apart from each other between the first lamp end 113 and the second lamp end 114.

The lamp 110 may be divided into a plurality of sections 115, 116, and 117. A section from the first lamp end 113 to a position spaced apart from the first lamp end 113 by a predetermined distance in the traveling direction Z may be defined as a first section 115. A section from the first section 115 to a position spaced apart from the first section 115 by a predetermined distance in the traveling direction Z may be defined as a second section 116. A section from the second section 116 to the second lamp end 114 may be defined as a third section 117. The first, second, and third sections 115, 116, and 117 may be integrally formed.

The plurality of pattern portions 112 may be formed in the respective first, second, and third sections 115, 116, and 117. The plurality of pattern portions 112 may be formed inside each of the sections 115, 116, and 117.

The plurality of pattern portions 112 are spaced at intervals D1, D2, and D3 which may become narrower away from the light source 163. The interval D1 between the plurality of pattern portions 112 in the first section 115 may be greater than the interval D2 between the plurality of pattern portions 112 in the second section 116. The interval D2 between the plurality of pattern portions 112 in the second section 116 may be greater than the interval D3 between the plurality of pattern portions 112 in the third section 117. Accordingly, the intervals between the plurality of pattern portions 112 become narrower away from the light source 163, thereby facilitating refraction or reflection of light at a position away from the light source 163. Accordingly, the light emitted from the light source 163 to the lamp 110 may be uniformly diffused in the longitudinal direction of the lamp 110 and may be uniformly projected in the longitudinal direction of the fan 15.

While the preferred embodiments have been particularly shown and described, the present specification shall not be limited to the particular embodiments described above, and it will be understood by an ordinary skilled person in the art that various changes in form and details may be made therein without departing from the scope of the present invention as defined by the following claims, and the alternative embodiments should not be individually understood from the inventive concept and prospect of the present disclosure.

Certain embodiments or other embodiments of the disclosure described above are not mutually exclusive or distinct from each other. Any or all elements of the embodiments of the disclosure described above may be combined with another or combined with each other in configuration or function.

For example, a configuration "A" described in one embodiment of the disclosure and the drawings and a configuration "B" described in another embodiment of the disclosure and the drawings may be combined with each other. Namely, although the combination between the configurations is not directly described, the combination is possible except in the case where it is described that the combination is impossible.

## Claims

1. An air conditioner comprising:
a case (10) including an inlet (21) and an outlet (22);
a heat exchanger (17) disposed inside the case (10);
a fan (15) disposed inside the case (10) and elongated in a direction of a rotation axis, RX;
a guide (30, 50) extending toward the outlet (22) in a rotation direction of the fan (15); and
a sterilizer (100) disposed on the guide (30, 50) toward the fan (15), and
wherein the sterilizer (100) includes a lamp (110) extending in a direction in which the fan (15) extends.

2. The air conditioner of claim 1, wherein the guide (30, 50) comprises:
a rear guide (50) extending in the rotation direction of the fan (15); and
a front guide (30) disposed downstream of the rear guide (50) and extending toward the outlet (22),
wherein the sterilizer (100) is disposed on the front guide (30).

3. The air conditioner of claim 2, wherein the sterilizer (100) is coupled to the front guide (30) to be detachable from the case (10).

4. The air conditioner of claim 2 or 3, wherein the front guide (30) comprises a device coupling portion (33) recessed in a direction away from the fan (15), and providing a space in which the sterilizer (100) is disposed,
and/or
wherein the sterilizer (100) comprises a lamp case (130) in which the lamp (110) is accommodated, and
wherein the sterilizer (100) is coupled to the front guide (30).

5. The air conditioner of any one of claims 2 to 4, wherein the sterilizer (100) and the front guide (30) extend in a direction of the rotation axis, RX, of the fan (15).

6. The air conditioner of any one of claims 2 to 5, wherein the sterilizer (100) comprises a case cover (140) defining a continuous surface with a surface of the front guide (30) that faces the fan (15).

7. The air conditioner of any one of claims 2 to 6, wherein the case (10) comprises:
a front wall (11) spaced apart from the fan (15),
wherein the front guide (30) comprises:
a first plate (31) disposed below the front wall (11) and the rear guide (50);
a first rib (34) protruding upward from the first plate (31); and
a second rib (35) protruding upward from the first plate (31) and spaced apart from the first rib (34),
wherein the front wall (11) and the rear guide (50) are inserted between the first rib (34) and the second rib (35).

8. The air conditioner of any one of claims 2 to 7, wherein the front guide (30) comprises a lower protrusion (37) protruding toward the outlet (22) and located lower than the sterilizer (100).

9. The air conditioner of any one of claims 2 to 5, wherein the sterilizer (100) comprises a case cover (140) defining a continuous surface with a surface of the rear guide (50) that faces the fan (15).

10. The air conditioner of any one of claims 2 to 9, wherein the rear guide (50) comprises a guide protrusion (53) protruding toward the fan (15) and located higher than the sterilizer (100).

11. The air conditioner of any one of claims 2 to 10, wherein the rear guide (50) comprises a plurality of guide protrusions (53) protruding toward the fan (15), and spaced apart from each other in a direction in which the fan (15) extends,
wherein the sterilizer (100) comprises holders (120) disposed at positions between the plurality of guide protrusions (53).

12. The air conditioner of any one of claims 1 to 11, further comprising a drain pan (40) disposed below the heat exchanger (17) and located lower than the sterilizer (100).

13. The air conditioner of claim 12, wherein the drain pan (40) comprises:
a base (41) disposed below the heat exchanger (17); and
a first drain pan wall (42) extending from the base (41) toward the sterilizer (100), and located lower than the sterilizer (100),
or
wherein the drain pan (40) comprises:
a first drain pan wall (42) extending toward the sterilizer (100); and
a drain pan protrusion (43) protruding from the first drain pan wall (42) in a direction away from the sterilizer (100).

14. The air conditioner of any one of claims 1 to 13, wherein the sterilizer (100) comprises a light source (163) connected to the lamp (110) and a light emitting portion (160) disposed on one side of the lamp (110),
and preferably
the air conditioner further comprising a fan motor (151) disposed on one side of the fan (15) and coupled to the fan (15),
wherein the light emitting portion (160) is disposed at a position corresponding to the fan motor (151).

15. The air conditioner of any one of claims 1 to 14, wherein the sterilizer (100) comprises a plurality of pattern portions (112) protruding toward a center of the lamp (110), and spaced apart from each other in a direction in which the lamp (110) extends, and preferably
wherein intervals at which the plurality of pattern portions (112) are spaced apart are set differently in a longitudinal direction of the lamp (110),
and preferably
wherein the sterilizer (100) comprises a light source (163) disposed on one side of the lamp (110) and connected to the lamp (110),
wherein the intervals between the plurality of pattern portions (112) become narrower away from the light source (163).
